(19)

**Europäisches Patentamt**
**European Patent Office**
**Office européen des brevets**

(11) **EP 4 523 682 A1**

(12) **EUROPEAN PATENT APPLICATION**

(43) Date of publication:
**19.03.2025 Bulletin 2025/12**

(21) Application number: **23382932.4**

(22) Date of filing: **13.09.2023**

(51) International Patent Classification (IPC):
**A61K 9/14** (2006.01)     **A61K 31/201** (2006.01)
**A61K 47/69** (2017.01)    **A61P 17/02** (2006.01)

(52) Cooperative Patent Classification (CPC):
**A61K 31/201; A61K 9/146; A61K 47/6951; A61P 17/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **FUNDACION UNIVERSITARIA SAN ANTONIO (UCAM)**
  **30107 Guadalupe (Murcia) (ES)**
• **Fundacion para la Formacion e Investigación Sanitarias (FFIS)**
  **30120 El Palmar (Murcia) (ES)**

(72) Inventors:
• **NICOLÁS VILLAESCUSA, FRANCISCO JOSÉ EL PALMAR (MURCIA) (ES)**
• **STERLING FÉREZ, JAVIER GUADALUPE (MURCIA) (ES)**
• **GABALDÓN HERNÁNDEZ, JOSÉ ANTONIO GUADALUPE (MURCIA) (ES)**
• **NAVARRO SÁNCHEZ, SERGIO GUADALUPE (MURCIA) (ES)**
• **LÓPEZ-MIRANDA GONZÁLEZ, SANTIAGO GUADALUPE (MURCIA) (ES)**

(74) Representative: **Díaz Pacheco, Maria Desamparados**
**MIO patentes & marcas**
**Avda. de Granada, S/N**
**Centro de Negocios Vega Plaza - Oficina 14**
**30500 Molina de Segura (Murcia) (ES)**

(54) **PROCESS TO COMPLEX OLEANOLIC ACID WITH CYCLODEXTRINS AND PRODUCTS OBTAINED THEREOF**

(57)     A process to complex oleanolic acid that comprises putting in contact oleanolic acid and at least one type of cyclodextrins in an aqueous mixture, dehydrate the product obtained in the previous step to obtain a solid powder comprising oleanolic acid/cyclodextrins complexes and the product obtained with said process, as well as its therapeutic use in skin disorders.

**EP 4 523 682 A1**

**Standard Curve OA**

$y = 200{,}93x$
$R^2 = 0{,}9997$

Fig.1

## Description

### Field of the Invention

The present invention relates to the field of biochemistry and cell biology, more particularly to a process to complex oleanolic acid with cyclodextrins and its use on normal and injured human skin.

### Background

Oleanolic acid (OA) is a particularly noteworthy molecule, as it is easier to obtain from plants than its two isomers, ursolic and maslinic acid (UA, AM), being present at different concentrations in the seed and pericarp of some fruits. The properties of OA make it a molecule with high therapeutic potential. In fact, one of its least studied properties is its promoting effect on wound healing. For this reason, the study of the effect of OA on wound healing, together with its improvement for application in wounds, are the main objectives of the proposed invention.

However, the use of OA in free form presents a number of limitations mainly due to its hydrophobic nature. Such molecules are poorly soluble in water, making the development of aqueous preparations significantly more difficult, in anticipation of administration to wounds. In addition to this, OA addition to aqueous solutions requires organic solvents such as dimethyl sulfoxide (DMSO), which have cytotoxic effects. In relation to this, the low solubility compromises the amount of OA taken up by epithelial cells and the beneficial effects on cell migration are limited, given its low availability to the cells. On the other hand, the use of OA in free (OA/DMSO) form requires it to be used in very low concentrations to achieve its healing-promoting effect, rapidly depleting the OA supply.

Due to these limitations, there is a need for new vehicles to improve the preservation, protection, delivery and effect of OA in wound healing.

Cyclodextrins (CD) are polymers composed of glucose units linked by $\alpha$-1,4-linkages which are produced from starch by an enzymatic process in plant cells. There are three forms of CDs: $\alpha$-CD, $\beta$-CD, and $\gamma$-CD.

The scientific article Lopez-Miranda S, Guardiola L, Hernandez-Sanchez P, Nunez-Delicado E. Complexation between oleanolic and maslinic acids with native and modified cyclodextrins. Food Chem. 2018;240:139-46 disclose encapsulations of oleanolic acid and maslinic acid with native cyclodextrins ($\alpha$-CD, $\beta$-CD and $\gamma$-CD) and their equivalents modified with hydroxypropyl groups (HP-$\alpha$-CD, HP-$\beta$-CD and HP-$\gamma$-CD).

However, this article does not disclose the complexation process of the present inventions and the advantages the final product has in comparison with the mentioned method. Particularly, this article does not disclose a dehydration step which make the final product more soluble and stable. The advantages of the process of the invention, and more concretely, the dehydration step, are the following:

- Better handling of the oleanolic acid

- Allows dissolving the complexes in solutions with osmolarity compatible with cell cultures, respecting their viability and biocompatibility.

- Allows the complexes to be dissolved in sterile solutions, thus allowing their use in cell cultures because it reduces the risk of contamination by bacteria or fungi.

- Better stability and preservation, as indicated in the study of different temperatures.

- It allows a better transport of the complexes as it does not necessarily require low temperatures.

### Description of the invention

In the present specification the terms "drying" "dehydration" and the grammatical variations thereof are synonyms and can be used interchangeably.

In the present specification the term "OA/DMSO" refers to free OA in water that requires the presence of dimethyl sulfoxide (DMSO), an organic solvent, to increase the water solubility of OA.

In the present specification the terms "OA encapsulation" and "OA complexation" and the grammatical variations thereof are synonyms and can be used interchangeably.

The first object of the invention relates to a process to complex oleanolic acid that comprises:

a) putting in contact oleanolic acid and at least one cyclodextrin in an aqueous mixture

b) dehydrate the product obtained in the previous step to obtain a dry powder comprising oleanolic acid/cyclodextrin complexes.

The term "Dehydration" means to reduce the water content. The reduction of the water content can be total or partial compared to the initial water content of the product of step a). Dehydration can be a reduction of the water content of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64,65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% compared to the initial water content of step a)

The reduction of the water content is an essential step in the process of the invention. The technical effect of this feature is that as it allows to increase the concentration of the complexes when used in order to see a therapeutic effect.

In a particular embodiment oleanolic acid and the cyclodextrin are put in contact with aqueous solution simultaneously or immediately one after another.

In another particular embodiment the aqueous mixture may comprise any liquid miscible with water, for example an alcohol, in any proportion that do not alter the aqueous character of the solution. In a preferred embodiment the aqueous mixture is pure water The complexes obtained in step a) are not useful for therapeutic uses as the OA concentration in the aqueous solution will not be high enough to achieve an effective amount in order to obtain a therapeutic effect.

By the term "effective amount" of the complexes as provided herein is meant a nontoxic but sufficient amount of the compound to provide the desired result. As will be pointed out below, the exact amount required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount can be determined by one of ordinary skill in the art using only routine experimentation.

The product obtained can be rapidly dissolved in a polar liquid such as water, and be used on *in vitro* assays with cell lines.

In a particular embodiment, the cyclodextrin is selected from one or more of the groups consisting of: α-cyclodextrin, β-cyclodextrin, and γ-cyclodextrin, preferably β-cyclodextrin, and γ-cyclodextrin.

In another particular embodiment the cyclodextrin comprises at least one hydroxypropyl group. This chemical groups are added to the free hydroxyl (-OH) from the glucose units, outside the cyclodextrin ring, on its surface. This modification confers extra water solubility.

In a preferred embodiment the cyclodextrin is hydroxypropyl-β-cyclodextrin or hydroxypropyl- γ-cyclodextrin.

In the present specification, the expression "oleanolic acid/cyclodextrin" can refer to both "oleanolic acid/cyclodextrin" and/or "oleanolic acid/hydroxypropyl-cyclodextrin". Furthermore, cyclodextrin may also refer to hydroxypropyl-cyclodextrin.

In a particular embodiment the ratio oleanolic acid/cyclodextrin in step a) is between approximately 1:50 to 1:500, preferably approximately 1:100 to 1:400, more preferably approximately 1:150 to 1:300.

In a preferred embodiment the ratio oleanolic acid/cyclodextrin is between approximately 1:200 to 1:280, more preferably 1:220 to 1:260, even more preferably approximately 1:250.

Routine concentrations for example would be approximately 25 to 100 mM of a cyclodextrin and 12.5 to 50 mg OA in 125 to 500 ml deionized water, preferably approximately 50 mM of a cyclodextrin and 25 mg OA in 250 ml deionized water, this is a ratio of 0.2/50 μM/mM oleanolic acid/cyclodextrin (1:250)

Later in the examples of the inventions it will be seen that ratios are given in μM/mM oleanolic acid/cyclodextrin. Thus, in a particular embodiment of the invention the oleanolic acid/cyclodextrin in step a) is between 0.05 μM to 70 μM of OA per 0.03 mM to 18 mM cyclodextrin and keeping a ratio of approximately 1:100 to 1:400.

This ratio is important to achieve an effective OA encapsulation ensuring encapsulation dynamics during the process of the invention.

The main advantage of the method of the invention is that upon obtaining dried complexes, very concentrated solutions can be achieved by dissolving the complexes in the appropriate medium (e.g. 1 gram in just 1 ml), which makes it easy to work with when adding the final concentration required for the assay. This is impossible to do with liquid complexes.

The ratio of the formed complexes oleanolic acid/cyclodextrin in step a) is between approximately 3:1 to 1:3, preferably approximately 2:1 to 1:2, more preferably approximately 1.5:1 to 1:1.5.

In a preferred embodiment the ratio oleanolic acid/cyclodextrin of the formed complexes is between approximately 1.2:1 to 1:1.2

The oleanolic acid/cyclodextrin are putting in contact in step a) for a time comprised between approximately 5 h and 5 days, preferably, approximately 10 h and 3 days, more preferably approximately 15 h and 2 days.

The temperature in step a) is comprised approximately between 5°C and 40°C, preferably between approximately 10°C and 30°C.

In a preferred embodiment, the oleanolic acid/cyclodextrin are put in contact for a time comprised between approximately 15 h and 2 days and a temperature comprised between approximately 10°C and 30°C.

Step a) needs to be protected from light exposure.

The dehydration of step b) is performed by freeze-drying, spray drying, vacuum stove and/or rotary evaporator, preferably freeze-drying, spray drying as vacuum stove or rotary evaporator are less up-to-date.

The freeze drying comprises subjecting the product of step a) to a temperature of less than -30 °C for an appropriate time, until the aqueous solution with the complexes is completely frozen, and them under a vacuum, subject said product to a temperature comprised between approximately -80°C and -20°C to obtain a dry powder.

In a particular embodiment, the freeze drying comprises subjecting the product of step a) to a temperature of less than approximately -70 °C for an appropriate time, until the aqueous solution with the complexes is completely frozen, and them under a vacuum, subject said product to a temperature comprised between approximately -60°C and - 40°C to obtain a dry powder, the required time until the water is completely removed can vary, it can be 1 day, 2 days, 3 days, 4 days or more.

In a particular embodiment, the the aqueous solutions with the complexes is subjected to a temperature of at least -10°C, -20°C, -30°C, -40°C, -50°C, -60°C, -70°C, -80°C, - 90°C, until said solution with the complexes is completely frozen.

In another particular embodiment, in the freeze dryer, a vacuum system and a temperature of -80 °C and -20°C, preferably -60°C and -40°C, even more preferably approximately -48°C, will be applied for three days until the water in which the complexes were dissolved is completely removed.

The spray drying comprises injecting the product of step a) into a circuit that sprays said product at a temperature between approximately 110°C and 210°C and then cool it to a temperature between approximately 40°C and 90°C to obtain a dry powder for a time between 10 min and 2 h.

In a particular embodiment, the temperature of the circuit is preferably 120°C to 200°C, more preferably 130°C to 190°C, and even more preferably 140°C to 180°C.

In another particular embodiment, the temperature to cool the solution with the complexes after the spray is between approximately 50°C and 80°C even more preferably 60°C to 70°C, for a time between 20 min and 1h.

The advantage of the spray drying technique compared to the freeze-drying technique is that dehydrates the complexes faster as there is no need to freeze them before. While freeze drying technique takes about 3 days to get the complexes dehydrated, spray drying only takes half an hour to obtain a completely dry powder. Generally speaking, spray drying is cheaper, faster and more energy efficient alternative to freeze drying, however, the spray drying stronger process using high temperatures could decrease OA activity.

The vacuum stove consists of reducing water boiling point (e.g. from 100 °C to 60 °C) by applying vacuum inside the stove. Thus, this technique eliminates most of the water content of the product. The disadvantage that this technique has is the high energy cost and time consuming, since it needs a long time period to remove 100 % of the water content. In the case of complexed OA, this procedure could also compromise OA activity due to the elevated temperature applied. This technique is close to the new ones: freeze drying and spray drying.

The rotary evaporator also applies heat and vacuum, together with a centrifugation that produces water evaporation. This procedure is used to concentrate products that are dissolved in a liquid, so it does not remove 100% of the water content. For this reason, is a procedure that may need to be complemented with freeze drying or spray drying.

Another object of the invention relates to a dry powder oleanolic acid/cyclodextrin complex obtained by the above-described process.

The dry powder oleanolic acid/cyclodextrin complex is a solid obtained after the dehydration step b) of the process of the invention.

Later in the examples of the invention it will be seen that this powder is stable over a long storage time.

In a particular embodiment, the cyclodextrin is selected from one or more of the groups consisting of: $\alpha$-cyclodextrin, $\beta$-cyclodextrin, and $\gamma$-cyclodextrin, preferably $\beta$-cyclodextrin, and $\gamma$-cyclodextrin.

In another particular embodiment the cyclodextrin comprises at least one hydroxypropyl group. This chemical groups are added to the free hydroxyl (-OH) from the glucose units, outside the cyclodextrin ring, on its surface. This modification confers extra water solubility.

In a preferred embodiment the cyclodextrin is hydroxypropyl-$\beta$-cyclodextrin or hydroxypropyl- $\gamma$-cyclodextrin.

In the present specification, the expression "oleanolic acid/cyclodextrin" can refer to both "oleanolic acid/cyclodextrin" and/or "oleanolic acid/hydroxypropyl-cyclodextrin"

Another object of the invention relates to a solution comprising the dry powder oleanolic acid/cyclodextrin complex which is diluted in an aqueous solution, which may be mixed any other liquids such as an alcohol or solid excipients dissolved on it.

The concentration of the dissolved dry powder oleanolic acid/cyclodextrin complex is higher than the maximum concentration that can be obtained in the step a) of the process of the invention.

The advantage of dissolving a dry powder oleanolic acid/cyclodextrin complex in an aqueous mixture (preferably water) is that high concentrations of said complex can be obtained,

In a particular embodiment, the concentration of the dissolved dry powder oleanolic acid/cyclodextrin complex in an aqueous mixture is at least 0.001 g/ml, or at least 0.01 g/ml 0.1 g/ml, or at least 0.5 g/ml, or at least 1g/ml, or at least 2 g/ml, or at least 3 g/ml, or at least 5 g/ml up to 10 g/ml. Preferably between 0.01 g/ml to 0.09 g/ml, more preferably 0.02 g/ml to 0.06 g/ml. So, the concentration of OA in the solution comprising the dry powder oleanolic acid/cyclodextrin complex and an aqueous mixture is approximately 25 mM. This is the therapeutically effective concentration of OA which is impossible to achieve unless the complexes are previously dehydrated.

Another object of the invention relates to the oleanolic acid/cyclodextrin complex, described above and/or obtained by the above-mentioned process for use in the treatment of skin disorders

In a particular embodiment, the skin disorders are one or more of the following: wound healing, scarring, eczema, psoriasis, acne, rosacea, ichthyosis, vitiligo, urticaria and seborrheic dermatitis. The complexes of the invention are also useful to prevent and/or trweat endothelial vascular disfunction, which is needed for skin regeneration.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from skin disorders, the terms "treatment" and "treating" refer to the medical management of a subject with the intent to cure, ameliorate, or stabilize skin disorders. In the context of a subject at risk of developing skin disorders, the terms "treatment" and "treating" refer to the medical management of a subject with the intent to prevent skin disorders.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described, the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

**Brief description of the figures**

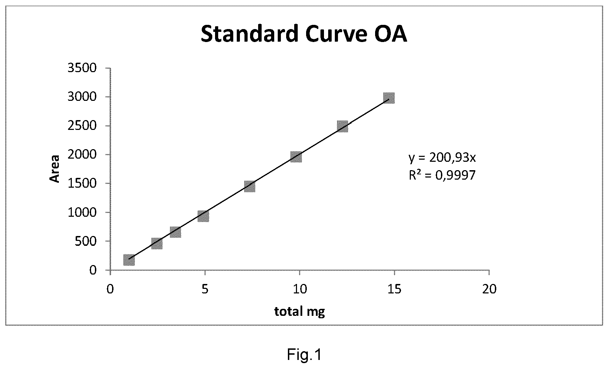

**Figure 1. Standard curve with OA by HPLC detection at 210 nm** (OA specific absorbance).

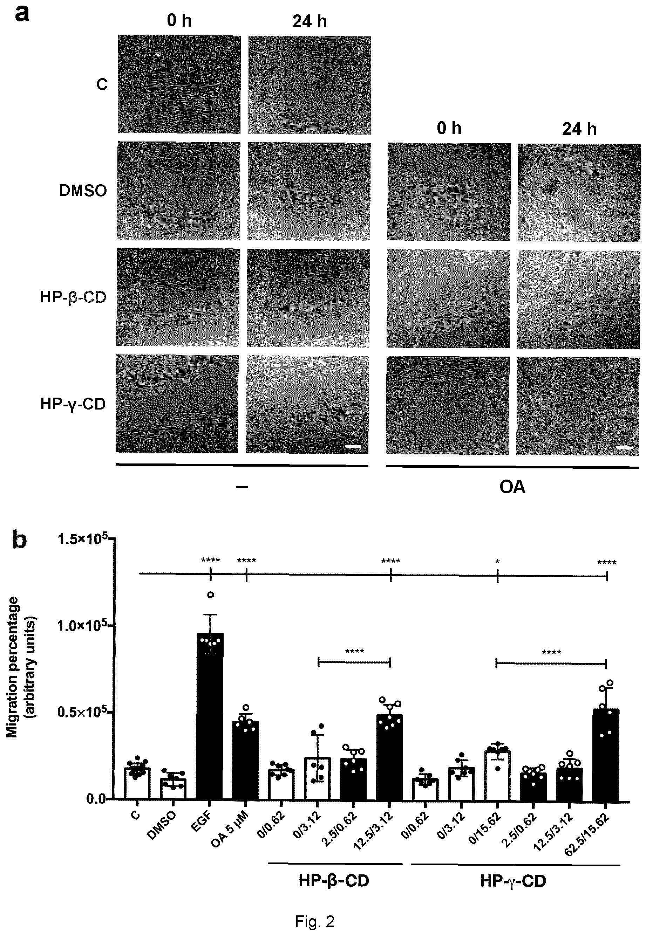

**Figure 2. Freeze dried OA/HP-$\beta$-CD and OA/HP-$\gamma$-CD complexes stimulate migration in Mv1Lu cells**. Confluent Mv1 Lu cells were scratched with a pipette tip and allowed to migrate for 24 hours. (a) Representative images of the wound healing assay with cell migration under basal conditions (C) compared to those with 5 $\mu$M OA/DMSO, 12.5/3.12 $\mu$M/mM OA/HP-$\beta$-CD and 62.5/15.62 $\mu$M/mM OA/HP-$\gamma$-CD; after 24h treatment. Equivalent concentrations of DMSO, HP-$\beta$-CD and HP-$\gamma$-CD were used as vehicle controls. Scale bar indicates 200 $\mu$m. (b) Plot represents cell migration as the difference between areas at time 0 hours and time 24 hours in each condition, named as migration percentage. X axis indicates treatment conditions, for those with HP-$\beta$-CD and HP-$\gamma$-CD, the concentrations are represented as molar ratio OA $\mu$M/CD mM. Epidermal growth factor (EGF) was added at 10 ng/ml as a positive migration control. Asterisks indicate statistically significant differences between conditions according to a One-way ANOVA statistical analysis (*p<0.05 and ****p<0.0001).

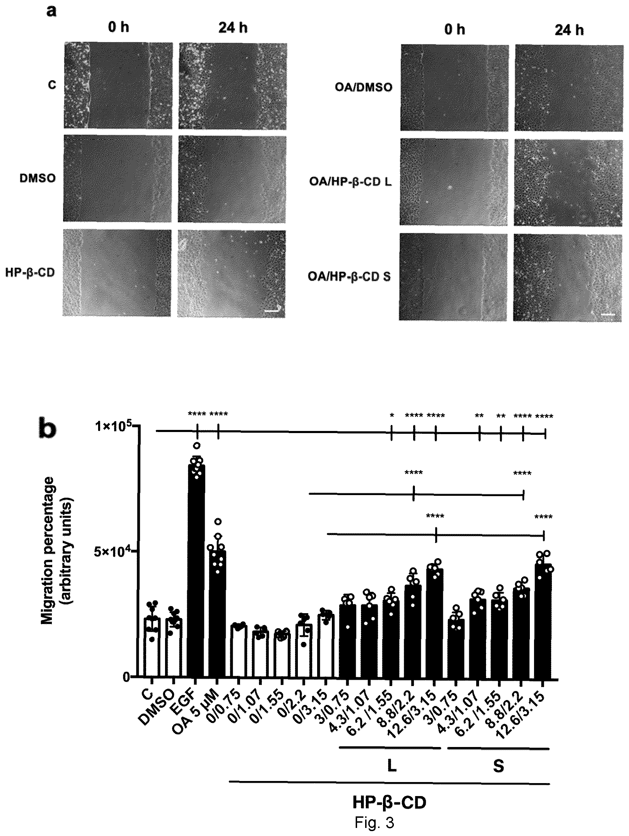

**Figure 3. Freeze dried and spray-dried OA/HP-β-CD complexes stimulate migration in Mv1Lu cells**. a) Representative images of the wound healing assay with cell migration under basal conditions (C) compared to those with 5 μM OA/DMSO, freeze dried (L) OA/HP-β-CD complexes and spray dried (S) OA/HP-β-CD, after 24h treatment. Equivalent concentrations of DMSO and HP-β-CD were used as vehicle controls. Scale bar indicates 200 μm. b) Plot represents cell migration as the difference between areas at time 0 hours and time 24 hours in each condition, named as migration percentage. X axis indicates treatment conditions, for those with HP-β-CD and HP-γ-CD, the concentrations are represented as molar ratio OA μM/CD mM. L (freeze dried); S (spray dried). Epidermal growth factor (EGF) was added at 10 ng/ml as a positive migration control. Asterisks indicate statistically significant differences between conditions according to a One-way ANOVA statistical analysis (*p<0.05, **p<0.005 and ****p<0.0001).

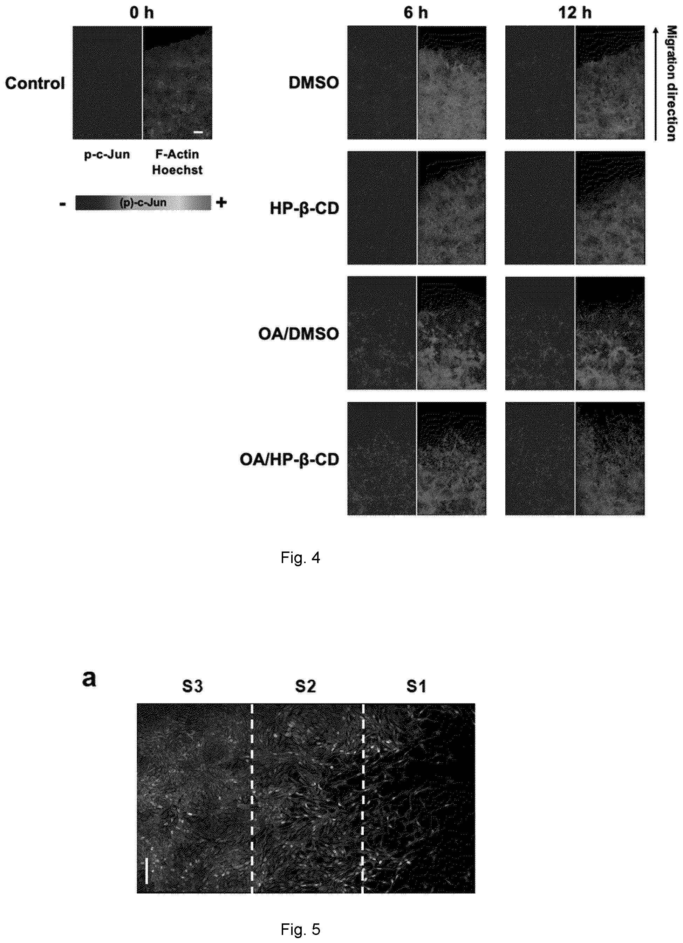

**Figure 4. OA/HP-β-CD complexes promote c-Jun phosphorylation at the edge of wound scratched Mv1Lu cells**. Confluent Mv1 Lu cells were scratched and allowed to migrate for the indicated times (6 and 12 hours). Cells were treated with 5 μM OA/DMSO or 12.5/3.12 μM/mM OA/HP-β-CD. Equivalent concentrations of DMSO and HP-β-CD were used as vehicle controls. Cells were immunostained with specific antibodies against c-Jun active phosphorylated form (p-c-Jun). Co-staining with phalloidin and Hoechst-33258 was used to show actin cytoskeleton and nuclei, respectively. Images of p-c-Jun fluorescence were converted into pseudo-color with imaged software to show the intensity of p-c-Jun staining. Actin fibers (F-actin): red. Nuclei: blue. Images were obtained with a confocal microscope. This experiment was repeated at least three times. Representative images are shown. Scale bar indicates 100 μm.

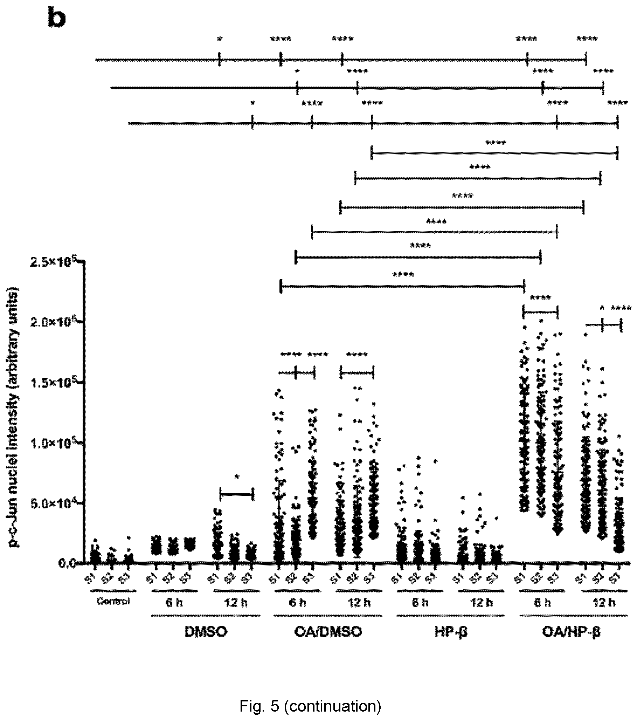

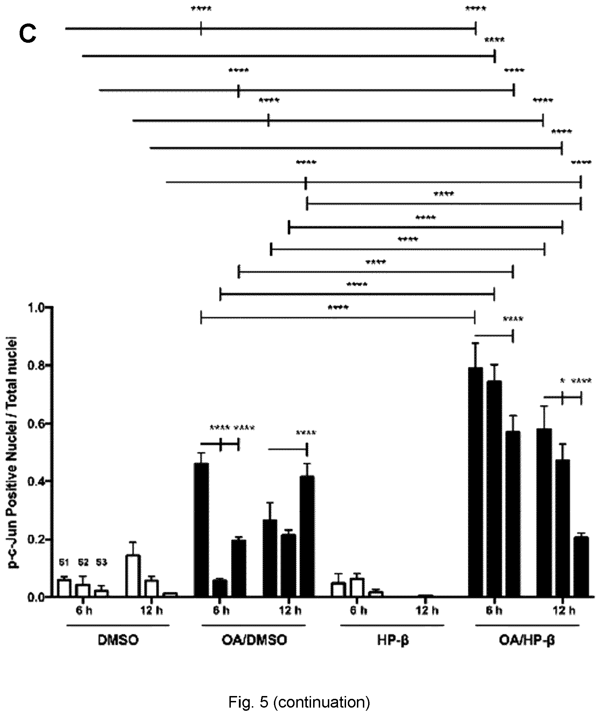

**Figure 5. OA/HP-β-CD complexes promote higher c-Jun transcription factor phosphorylation than OA/DMSO at wound edge**. (a) The image represents how a Tile Scan image is divided in three equal sectors for better analyze active c-Jun (p-c-Jun) expression. Scale bar indicates 100 μm. (b) Plot represents the p-c-Jun intensity in cell nuclei. Each point represents the intensity of p-c-Jun intensity in one nucleus, (c) Plot represents the relation between the number of positive p-c-Jun nuclei and total number nuclei existent in each sector. In order to exclude negative p-c-Jun nuclei, an intensity p-c-Jun threshold was set using the p-c-Jun intensity mean in basal (control) condition.

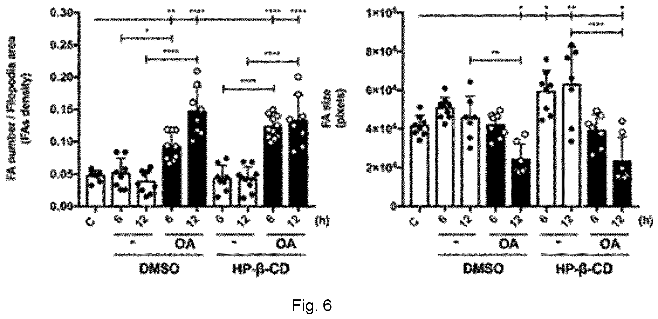

**Figure 6. OA/HP-β-CD complexes promote changes in focal adhesions (FAs) revealed by Paxillin**. Confluent Mv1Lu cells were scratched and allowed to migrate for 6 and 12 hours. Cells were treated with 5 μM OA/DMSO or 12.5/3.12 μM/mM OA/HP-β-CD. Equivalent concentrations of DMSO and HP-β-CD were used as vehicle controls. Cells were immunostained with specific antibodies against paxillin Co-staining with phalloidin and Hoechst-33258 was used to show actin cytoskeleton and nuclei, respectively (Not shown). Quantification of the density of FA (as FA number per filopodia area). FA size (average size) at the filopodia area. A One-way ANOVA statistical analysis was performed (*p<0.05, **p<0.005 and ****p<0.0001)

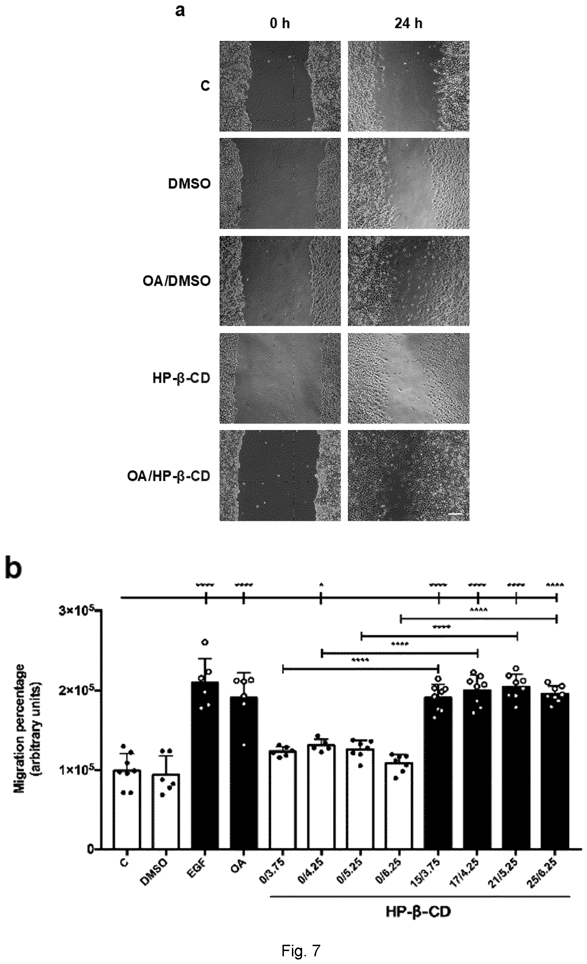

**Figure 7. OA complexes with modified cyclodextrins HP-β-CD promote cell migration in MDA-MB-231 cells**. Confluent MDA-MB-231 cells were scratched with a pipette tip and allowed to migrate for 24 hours. (a) Representative images of the wound healing assay with cell migration under basal conditions (C) compared to those with 5 μM OA/DMSO, 21/5.25 μM/mM OA/HP-β-CD and equivalent concentrations of DMSO or HP-β-CD after 24h treatment. Scale bar indicates 200 μm. (b) Plot represents cell migration as the difference between areas at time 0 hours and time 24 hours in each condition, named as migration percentage. X axis indicates treatment conditions, for those with HP-β-CD the concentrations are represented as molar ratio OA μM/CD mM. Epidermal growth factor (EGF) was added at 10 ng/ml as a positive migration control. Asterisks indicate statistically significant differences between conditions according to a One-way ANOVA statistical analysis (*p<0.05 and ****p<0.0001).

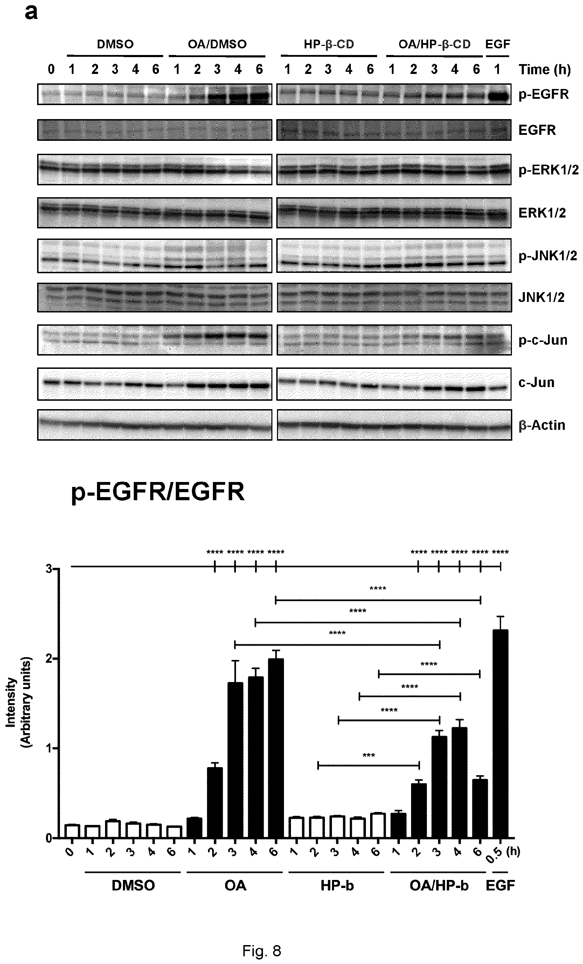

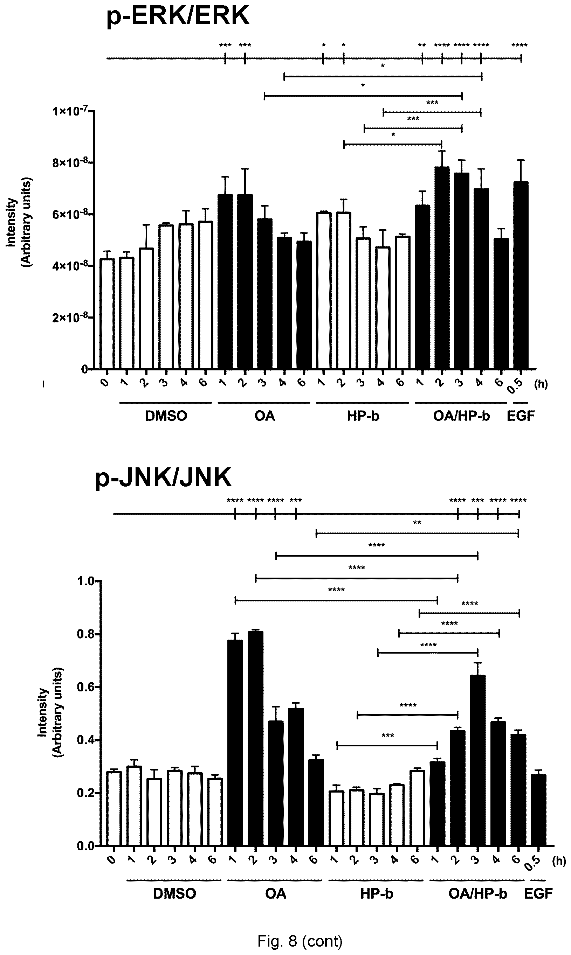

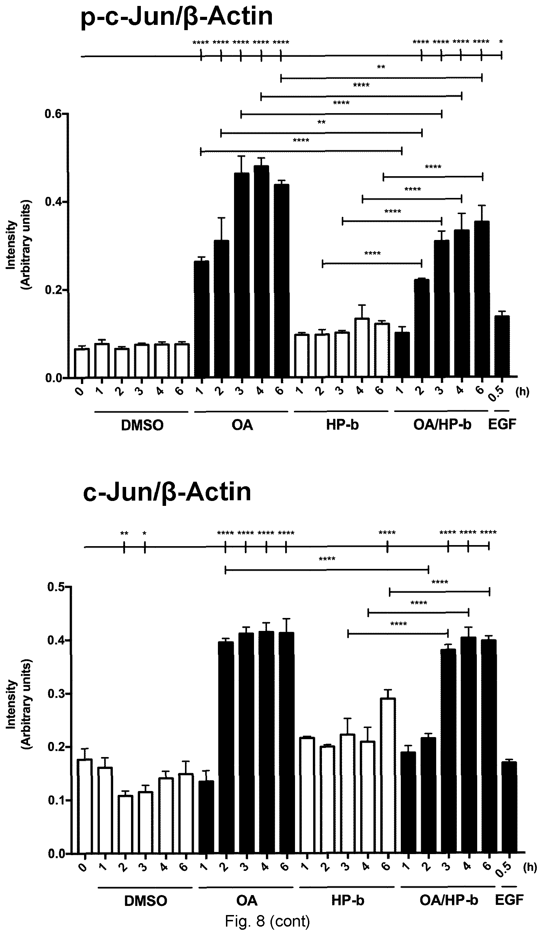

**Figure 8. Signaling pathways regulated by EGFR and c-Jun activation, necessary for cell migration, are induced by OA/HP-β-CD complexes in MDA-MB-231 cells.** (a) Total protein extracts from serum-starved sub-confluent MDA-MB-231 cells treated with 10 μM OA/DMSO, 21/5.25 μM/mM OA/HP-β-CD or 10 ng/ml EGF. These extracts were assayed at the indicated times (hours) targeting: phospho-EGFR (Tyr 1068), phospho-ERK1/2 (Thr 202/Tyr 204), phospho-JNK1/2 (Thr 183/Tyr 185) and phospho-c-Jun (Ser 63). Total protein expression was assayed for the above-mentioned active protein forms: EGFR, ERK1/2, JNK1/2 and c-Jun. β-Actin was used as a loading control. DMSO and HP-β-CD equivalent concentrations were added as vehicle controls. A representative experiment was shown. (EGFR, epidermal growth factor receptor; ERK1/2, extracellular signal-regulated kinases 1 and 2; c-Jun N-terminal kinases 1 and 2). (b) Column bar graphs represent intensity values of each protein assayed by Western blot, by collecting the data of three independent experiments. Intensity values were quantified and gathered by ImageJ software. Asterisks indicate statistically significant differences between the selected conditions according to a One-way ANOVA statistical analysis: (*p<0.05, **p<0.005, ***p<0.001 and ****p<0.0001).

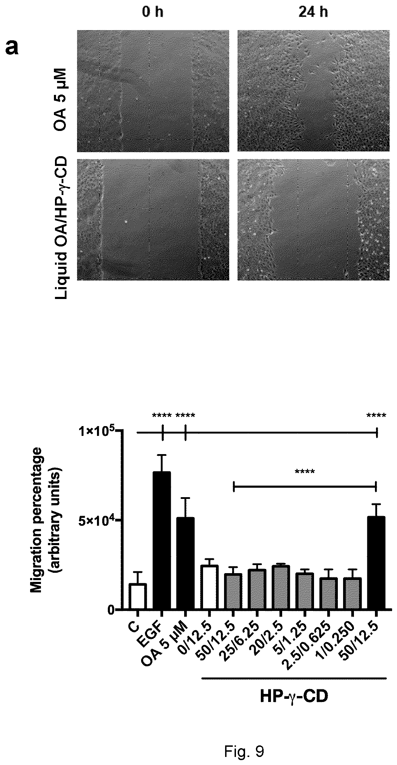

**Figure 9. Liquid OA/CDs complexes do not have the same effectiveness on cell migration as dehydrated OA/CDs complexes in Mv1Lu cells.** Confluent Mv1 Lu cells were scratched with a pipette tip and allowed to migrate for 24 hours. (a) Representative images of the wound healing assay with cell migration under 5 $\mu$M OA/DMSO compared to those liquid (no dehydrated) OA/HP-$\gamma$-CD complexes after 24h treatment. DMSO, and 0/12.5 $\mu$M/mM HP-$\gamma$-CD were used as vehicle controls. (b) Plot represents cell migration as the difference between areas at time 0 hours and time 24 hours in each condition, named as migration percentage. X axis indicates treatment conditions, for those with HP-$\gamma$-CD, the concentrations are represented as molar ratio OA $\mu$M/CD mM. Liquid OA/HP-$\gamma$-CD complexes concentrations used are represented in grey bars. Freeze dried 50/12.5 $\mu$M/mM OA/HP-$\gamma$-CD complexes were used as positive control. Epidermal growth factor (EGF) was added at 10 ng/ml as a positive migration control. Asterisks indicate statistically significant differences between conditions according to a One-way ANOVA statistical analysis (****p<0.0001).

## Examples

### Materials and Methods

**EXAMPLE** 1: COMPLEXATION OF OLEANOLIC ACID WITH CYCLODEXTRINS

Preparation of liquid oleanolic acid/cyclodextrin complexes.

The formation of liquid oleanolic acid/cyclodextrin complexes was performed as described in Lopez-Miranda 2018. Briefly, Excess amounts of OA was added to 10 ml of aqueous solutions of increasing concentrations from 0 to 13 mM for b-CDs and 0 to 50 mM for a-, g-, HP-a-, HP-b- and HP-g-CDs. The different phase solubility diagrams were prepared in glass test tubes and maintained in an ultrasonic bath (Ultrasons H.P. Selecta, Spain) for 60 min to reach equilibrium. The effect of temperature on the complexation process was studied by developing solubility experiments in distilled water at different temperatures (4 °C, 25 °C and 65 °C). The effect of pH on the complexation process was studied by developing solubility diagrams in buffered solutions of CDs at pH 3.0 (sodium acetate buffer), 6.5 (sodium phosphate buffer) and pH 9 (sodium borate buffer). After 60 min of ultrasound treatment, solutions were centrifuged in a microcentrifuge (Eppendorf Centrifuge S415D, Germany) at 10000 xg for 10 min and filtered using 0.45 $\mu$m nylon membrane filters (Chromafil. Macherey-Nagel, Germany).

Given the need to standardize the conditions of pH and temperature for the encapsulation step and further assay oleanolic acid/cyclodextrins on cell cultures, a condition with 25 °C and pH 7.0 was stablished since, as indicated below, they are the preferred parameters to obtain high encapsulation efficiency (EE) values.

On the other hand, given its better performance for oleanolic acid encapsulation and solubility, the modified hydroxypropyl beta and gamma (HP-$\beta$- and HP-$\gamma$-) cyclodextrin (CD) are chosen. They are purchased from Winplus International Limited (Ningbo, China). OA and cyclodextrins are added to 250 mL of aqueous solutions with a molar ratio of 0.2/50 (OA/CD) for both HP-$\beta$- and HP-$\gamma$-CDs. These molar proportions were selected according to phase solubility diagrams previously published by López-Miranda et al. (2018), since they formed type 1:1 complexes, one OA encapsulated molecule per one cyclodextrin molecule. Solutions are constantly stirred during 24 h at 20 °C in the dark for complexes formation in said aqueous solutions.

Oleanolic acid/cyclodextrin (OA/CDs) complexes dehydration.

The aim of this step is to prepare an anhydrous presentation, i.e. a solid powder preparation with the formed OA/CDs complexes. This dehydration is necessary for testing the mentioned complexes in an *in vitro* model of epithelial cell cultures. The advantage of a dehydrated complex is that it allows its easy dissolution in the culture media used to grow the cells, thus allowing the preparation of solutions at the desired concentration of OA/CDs. This culture media is an aqueous solution.

After Solutions were constantly stirred during 24 h at 20 °C in the dark, OA/CD solid complexes are obtained by dehydrating 250 mL volume of dissolved complexes. The recovered dehydrated solid complexes are stored in plastic containers protected from light for posterior analysis and characterization. This samples, already with encapsulated OA, will be resuspended with ultrapure water and re-injected into a HPLC for OA detection and quantification. Previously, a standard curve is elaborated with pure OA to better quantify OA complexes solutions. HPLC allows the detection and quantification of a given compound by knowing its absorbance at a specific wavelength ($\lambda$). It consists of injecting a solution of the compound, in this case OA, to drive it through a mobile phase in the HPLC machine. The encapsulated OA solution is injected into the HPLC for detection and quantification of the OA. Due to the chemical structure of OA, it has a specific absorbance and can therefore be detected at a reference wavelength of 210 nm. Furthermore, using the absorbance peak at 210 nm recorded by the HPLC machine by the detection of OA, the amount of OA in solution can be calculated by determining the peak area.

<u>OA/HP-β-CD and OA/HP-v-CD complexes drying by freeze drying and spray drying methods</u>

The formed OA/HP-β-CD complexes were subjected to drying by freeze drying or spray drying. First, for the freeze-drying method, the aqueous solutions with the complexes OA/HP-β-CDs and OA/HP-γ-CDs shall be subjected to -80°C to freeze them completely. Then, in the freeze dryer, a vacuum system and a temperature of -48°C will be applied for three days until the water in which the complexes were dissolved is completely removed. Secondly, the Spray Dry technique dehydrates the complexes in less time and without the need to freeze them beforehand. In this case, the solution with the complexes is injected into a circuit that sprays it at a temperature of 170°C, eliminating the water from the solution and then cooling it to 68°C. In just half an hour, the dried complexes are obtained in a dry powder form.

<u>Evaluation of OA concentration in the complexes and measurement of their stability at different temperatures at different times</u>

To determine the amount of encapsulated OA present in the powder preparations obtained by freeze drying or spray drying, several samples of the powder preparations were reconstituted with ultrapure water, in order to be able to detect the OA by HPLC, as described above. Considering the amounts of OA before and after encapsulation (mg of OA in the initial solution), together with the new data of OA in the powdered preparations (mg of OA in solid complex and total g of complexes), the encapsulation efficiency (EE) and the OA load in the complexes (DL) are calculated according to the following formulae:

$$EE\ (\%) = \frac{\text{total compound encapsulated in solid complex (mg)}}{\text{total initial compound in solution (mg)}} \cdot 100$$

$$DL\ (mg\ g^{-1}) = \frac{\text{total compound encapsulated in solid complex (mg)}}{\text{total weight of solid complex (g)}}$$

In order to study the stability and conservation of the powdered complexes over time. Sais OA/CD complexes were stored at three different temperatures (20°C, 4°C and -80°C) and were analyzed later to quantify the encapsulated OA by HPLC (days and weeks after complexation).

**EXAMPLE 2: FUNCTIONAL TESTING OF THE COMPLEXES OF THE INVENTION**

The complexes of the invention were tested in functional assays with epithelial cell models. In these assays, the promoter effect of OA on cell migration and the expression of proteins necessary for this phenomenon was evaluated.

<u>Cell culture</u>

Mink Lung Epithelial (Mv1Lu) cells are grown in Eagle's Minimum Essential Medium (EMEM) (Biowest, Nuaillé, France) supplemented with 10% Fetal Bovine Serum (FBS, Gibco, Thermo Fisher Scientific, Rockford, IL, USA), 1% Penicillin/Streptomycin and 1% L-Glutamine (both from Biowest, Nuaillé, France). Mv1Lu cells are cultured in an incubator at 37 °C with a 5% $CO_2$ humified atmosphere. Human mammary gland cells (MDA-MB-231) are grown in Dulbecco's Modified Eagle Medium (DMEM) supplemented as mentioned above for EMEM. MDA-MB-231 cells are incubated in an incubator at 37 °C with a 7.5% $CO_2$ humified atmosphere.

<u>Wound-healing scratch assay on epithelial cell lines with OA/CDs complexes to measure their biological activity on cell migration</u>

Mv1Lu or MDA-MB-231 are seeded in 24-well plates until they reach 100% confluence, using the appropriate medium for each line with all supplements. At that time, medium is changed to FBS-free medium for 24 hours. At the initial time (T0), a cross-shaped scratch is made on the cell monolayer using a sterile p-200 μl pipette tip. After replacing FBS-free culture medium to wash out released cells, OA, OA/HP-β-CD or OA/HP-γ-CD (the concentration used in each assay is indicated at each figure legend) are added. An equivalent volume of DMSO, HP-β-CD and HP-γ-CD is added. In parallel, a positive control is set by adding 10 ng/ml epidermal growth factor (EGF, Sigma-Aldrich, St Louis, MO, USA). Additionally, pharmacological inhibitors against key proteins on migration regulation are added to OA conditions: 2.5 μM PD153035 (Epidermal Growth Factor inhibitor, EGFRi), 50 μM PD098059 (Mitogen-activated protein kinase kinase inhibitor, MEKi) and 15 μM SP600125 (c-Jun N-terminal kinase inhibitor, JNKi). After a 24-hour incubation period, cells are fixed with 4% formaldehyde (Applichem GmbH, Darmstadt, Germany) in PBS (Biowest, Nuaillé, France) for 10 minutes. Finally, well plates are washed twice with PBS. Pictures are taken at 10X magnification using an optical microscope equipped with a digital camera (Motic Optic AE31, Motic Spain, Barcelona, Spain). To quantify cell migration, the areas of the gaps in the wounds are measured by imaged software. The initial cell-free surface (time 0 h) is subtracted from the endpoint cell-free surface (time 24 h) and plotted in a graph.

Cell-front migration assay, subcellular localization assay

Mv1Lu cells are grown until they reach confluence on round glass-coverslips in 10% FBS-EMEM medium. At this time, cells are washed and a 24h serum-starvation period is set by replacing the medium with FBS-free EMEM. After this, the epithelium is scratched using a razor blade, which produces an artificial wound with enough space to allow cells to migrate. The newly wound is set as a time 0 hours of the experiment and then 5 μM OA/DMSO or 12.5/3.125 μM/mM OA/HP-β-CD or are added to the medium. As parallel conditions, DMSO or HP-β-CD (3.125 mM) equivalent volumes are added as vehicle controls. After the selected times of incubation, glass-coverslips are fixed with 4% formaldehyde (Applichem GmbH, Darmstadt, Germany) in PBS (Biowest, Nuaillé, France) for 10 minutes and washed twice with PBS. After this, cells are permeabilized with 0,3% Triton X-100 (Sigma-Aldrich, St Louis, MO, USA) in PBS for 10 minutes. For immunostaining, a blocking is performed for 30 minutes at room temperature in PBS solution with 10% FBS, 5% skim milk (Beckton Dickinson, Franklin Lakes, NJ, USA), 0,3% bovine serum albumin (BSA, Sigma-Aldrich, St Louis, MO, USA) and 0,1% Triton X-100. Then, cells are incubated 1 hour at room temperature with anti-paxillin or anti-phospho-c-Jun antibodies, diluted in blocking solution without skim milk. Proper fluorescent-labelled secondary antibodies (see Antibodies section) are co-incubated for 30 minutes with Alexa Fluor 594 conjugated phalloidin (Molecular Probes, Thermo Fisher Scientific, Waltham, MA, USA), and Hoechst 33258 (Fluka, Biochemika, Sigma-Aldrich, St Louis, MO, USA) to reveal actin cytoskeleton and nuclei respectively. Once samples are inmunostained, image acquisition is performed with a confocal microscope (LSM 510 META from ZEISS, Jena, Germany). The setting of images was performed using Zeiss Efficient Navigation (ZEN) interface software (ZEISS, Jena, Germany). When a wider view of the migration front is required, concretely in phospho-c-Jun staining (indicated in Figures), 4X3 linked fields are acquired by the "Tile scan" ZEN tool. Subsequently, tile scans fluorescent signals are converted to a linear mode and covered with a full data range using the Rainbow look up table (Rainbow LUT) in Image J software. In order to quantify phospho-c-Jun levels in immunofluorescence pictures, images are analyzed and quantified by Image J software. For this purpose, pictures in 8-bit three-channel format (Red, Green, Blue, RGB) were divided into three separate color channels (three pictures). By using blue channel picture (Hoechst staining), regions of interest (ROIs) are stablished to define each nucleus, creating as many ROIs masks as nuclei in the image. Then, by overlapping these masks onto the corresponding green channel picture (p-c-Jun staining), we calculate the green intensity value of each nucleus (ROI). Because of the large area covered in each picture (Tile scan), they are divided in three equal sectors (S1, S2 and S3), being S1 the outermost edge on the wound. Within each sector, the quantified signal of each nucleus is used as a replicate to obtain p-c-Jun intensity data in each of the conditions performed. For a better understanding, the relation of number of p-c-Jun positive nuclei between total nuclei number is calculated. For this purpose, a basal p-c-Jun intensity mean is calculated considering the control condition of the assays, set as a threshold. In this sense, nuclei with p-c-Jun intensity over the threshold are set as positive nuclei. On the other hand, "Z stack" ZEN feature is used when deep cytoskeleton structures observance is required, taking a proper number of pictures along the Z axis. Focal adhesion (FA) quantifications are performed as described in Horzum et al., 2014 by using CLAHE and Log3D macros for ImageJ {Horzum, 2014 #553}. Briefly, focal adhesions are quantified from paxillin stained acquired pictures. We used three different images for each condition. Specifically, cell filopodia are selected as regions of interest (ROIs) and the resulting areas (containing FAs) are considered for further analysis. A number of five filopodia are considered from each picture. Then, the number of FAs are calculated in each filopodia by using the previously mentioned macros. The obtained number is divided by the total filopodia area to determine FAs density. In parallel, the size of each FA is measured using the macros mentioned above. Finally, FA average size is calculated for each treatment.

Western blot

Mv1Lu or MDA-MB-231 cells are seeded and allowed to reach 60% confluence in 10 cm diameter plates. At this time, culture medium containing 10% FBS is replaced by an FBS-free medium, incubating the cells for a 24-hours period. Serum-deprived cells are treated with either OA, OA/HP-β-CD, DMSO, HP-β-CD or 10 ng/ml EGF. After time incubations, cells are harvested, washed twice with ice-cold PBS and lysed with 20 mM TRIS pH 7.5, 150 mM NaCl, 1 mM EDTA, 1.2 mM MgCl2, 0.5%, Nonidet p-40, 1mM DTT, 25 mM NaF and 25 mM β-glycerophosphate supplemented with phosphatase inhibitors (I and II) and protease inhibitors (all from Sigma-Aldrich, St Louis, MO, USA). Total protein amount of all extracts is measured and normalized by Bradford assay (Sigma-Aldrich, St Louis, MO, USA). The extracts are analyzed by SDS-PAGE followed by Western blot (WB) using the indicated antibodies (see Antibodies section). Blots are revealed by using Horseradish peroxidase substrate (ECL) (GE Healthcare, GE, Little Chalfont, United Kingdom) and images are taken with a Chemidoc XRS1 (Bio-Rad, Hercules, Ca, USA). For protein quantification, Western pictures in 8-bit format are processed in imaged software. Subsequently, in all Western blot pictures, a lane is stablished for each of the samples. In each lane, the band is selected according to the specific size (kDa) of the protein of interest. For each total protein and their phosphorylated version, each band's intensity peak is plotted, and next, the area under the plot is measured by using "Wand (tracing) tool" of imaged to obtain the intensity value. In order to normalize, obtained intensity values are referred to obtained intensity values of either the unphosphorylated form of the protein (total) or a loading control protein such as β-actin, only if the unphosphorylated form is undetectable (non-available antibody for detecting the unphosphorylated form).

Antibodies

The following commercial primary antibodies are used: anti-phospho-ERK1/2, anti ERK1/2, anti-JNK1/2, anti-phospho-JNK1/2 and anti-phospho-c-Jun (all from Cell Signaling Technology, Danvers, MA, USA); anti-phospho-EGFR (Thermo Fisher Scientific, Rockford, IL, USA); anti-EGFR, anti-paxillin and anti-c-Jun (Santa Cruz Biotechnology, Heidelberg, Germany); and anti-β-actin (Sigma-Aldrich, St Louis, MO, USA). Secondary antibodies are anti-rabbit IgG Horseradish peroxidase linked F(ab')2 I fragment (from donkey) (GE Healthcare, GE, Little Chalfont, United Kingdom), anti-mouse IgG$_1$ (BD Pharmingen, Beckton Dickinson, Franklin Lakes, NJ, USA); Alexa Fluor 488 conjugated anti-mouse (from donkey) (Thermo Fisher Scientific, Rockford, IL, USA).

Statistical analysis

All the collected data are analyzed using Graph Pad Prism 7 software. In every analysis, classic statistical parameters were calculated and statistical tests are performed with a 95% confidence interval, consequently, P-values lower than 0.05 are considered to be statistically significant. At the figure legends, the asterisks denote statistically significant difference between conditions (*p<0.05, **p<0.005, ***p<0.001 and ****p<0.0001). Data of intensity values, collected from Western blots, are analyzed by a One-way ANOVA test, comparing the mean of each condition with the mean of every other condition. Then, a Tukey's multiple comparisons test is performed.
Data of intensity values obtained from p-c-Jun nuclei quantifications in immunofluorescence pictures are analyzed by using two statistic tests: Two-way ANOVA test and One-way ANOVA test. Concretely, a Two-way ANOVA following Tukey's multiple comparison test is performed to compare the p-c-Jun intensity mean between sectors from different conditions (e.g. S1 DMSO versus S1 OA). On the other hand, a One-way ANOVA, following Tukey's multiple comparison test is performed to compare p-c-Jun intensity mean between sectors from the same condition (e. g. S1 DMSO versus S2 DMSO).

**Results**

Example 1.1: Analysis and quantification of OA by high-performance liquid chromatography (HPLC)

HPLC allows to detect and quantify OA in a solution on the basis of its absorbance at a specific wavelength (210 nm). Commercial OA from Sigma with a purity of 98% was used in this experiment. In order to obtain an adequate quantification of the OA /cyclodextrin complexes, a standard curve of the OA to be used is prepared. To do this, the HPLC equipment must be calibrated using a column specially designed for organic molecules and a mobile phase of acetonitrile, acetic acid and water. A solution of OA at a concentration of 0.5 mg/ml in methanol is then prepared in such a way that the HPLC equipment takes different volumes (punctures) of this solution in increasing order (Table **1**). In this way the absorbance peaks of OA at 210 nm are recorded and their area is calculated, which is directly proportional to the volume of the spike. This provides a standard curve on which to base subsequent quantifications. Once the equation for the standard curve has been calculated, the amount of OA in the test solution can be calculated from the recorded absorbance of the OA (**Figure 1**).

Table 1. OA quantifications to produce the standard or calibration curve.

| Injected μL | OA concentration mg/mL | Area (210nm) | Total μg |
|---|---|---|---|
| 2 | 0.5 | 181 | 0.98 |

(continued)

| Injected μL | OA concentration mg/mL | Area (210nm) | Total μg |
|---|---|---|---|
| 5 | 0.5 | 464.9 | 2.45 |
| 7 | 0.5 | 656.8 | 3.43 |
| 10 | 0.5 | 935.2 | 4.9 |
| 15 | 0.5 | 1451 | 7.35 |
| 20 | 0.5 | 1960.9 | 9.8 |
| 25 | 0.5 | 2489.1 | 12.25 |
| 30 | 0.5 | 2978.5 | 14.7 |

Oleanolic acid/cyclodextrin complexation

Beta- and gamma-cyclodextrins modified with hydroxypropyl groups (CD-HP-β and CD-HP-γ respectively) from Winplus International Limited are used for OA conjugation. In general, aqueous solutions of 50, 250 or 500 ml are prepared to which OA and cyclodextrins (CD-HP-β or CD-HP-γ) are added in a molar ratio of 0.2:50 (oleanolic acid:cyclodextrin). This molar ratio allows the encapsulation of one OA molecule for each cyclodextrin molecule, so that OA/CD complexes are formed in a 1:1 ratio. The OA/CD solutions are stirred for 24 hours at room temperature and protected from light. After 24 hours the solutions are sampled to determine the amount of encapsulated OA by extrapolation to the standard curve. **Table 2** shows representative data from one of the OA encapsulation batches performed with HP-β-CDs and HP-γ-CDs.

Table 2. Quantifications of OA in a solution with CDs after 24 hours of stirring.

| CD | CD g (50mL) | OA mg (50mL) | Liquid Complex Area | Liquid Complex Area (Avge) | mg/ml | Total mg. Liquid Complex |
|---|---|---|---|---|---|---|
| CD-HP-β | 3,85 | 5,0 | 314,4 | 315,3 | 0,08 | 3,92 |
| | | | 314,1 | | | |
| | | | 317,5 | | | |
| CD | CD g (50mL) | OA mg (50mL) | Liquid Complex Area | Liquid Complex Area (Avge) | mg/ml | Total mg. Liquid Complex |
| CD-HP-y | 4,4 | 5,0 | 322,6 | 319,7 | 0,08 | 3,98 |
| | | | 319,9 | | | |
| | | | 316,6 | | | |

CDs with β- and γ-type hydroxypropyl (HP) groups gave similar OA loadings and encapsulation yields (between 80 and 100 %).

Example 1.2: Dehydration of OA/HP-β-CD complexes by freeze-drying and spray-drying methods

The OA/CDs solutions were divided into equal volumes to undergo two different dehydration processes: freeze-drying and spray-drying. For freeze-drying, the OA/CDs solutions were first subjected to lyophilization in a Christ Alpha 1-2 LD Plus freeze dryer under vacuum at a temperature of -48°C. On the other hand, the OA/CDs complexes were dried in a Spray-Dry Buchi B-290 at a temperature of 170°C. In order to determine the efficiency of each of the two processes, the dehydration yield (RD), a percentage that indicates how many grams of CDs were recovered in the dehydrated powder relative to the initial grams of CDs weighed before OA encapsulation, was calculated. These data are presented in Table 3.

$$RD\ (\%) = \frac{\text{solid complexes obtained (g)}}{\text{total solids in solution (g)}} \cdot 100$$

Table 3. Calculation of freeze-drying and Spray-Dry dehydration yields.

| Freeze drying | | | |
|---|---|---|---|
| CD | Initial CD g | CD g powder after FD. | RD (%) |
| HP-β-CD | 7,7 | 7,17 | 93,1 |
| HP-γ-CD | 8,8 | 8,03 | 91,3 |
| Spray drying | | | |
| CD | Initial CD g | CD g powder after SD. | RD (%) |
| HP-β-CD | 7,7 | 6,72 | 87,3 |
| HP-γ-CD | 8,8 | 6,23 | 73,29 |

As can be seen in **Table 3,** the dehydration yields obtained by Spray Dry were slightly lower than those obtained by Freeze drying. The speed of the Spray Dry treatment seems to affect the amount of OA/CDs powder recovered, but this small loss is acceptable as long as the encapsulated OA does not lose its effect on cell migration in subsequent in vitro tests on epithelial cells (see following sections) which is equally effective.

Example 1.3: Evaluation of OA concentration in the complexes and measurement of their stability

Once the solid (powdered) OA/CDs complexes have been obtained, the encapsulation yields of each type of cyclodextrin (EE) and the loading of active material (DL), i.e. OA, per gram of cyclodextrin must be calculated. To do this, the following calculations are carried out:

$$\text{EE (\%)} = \frac{\text{total compound encapsulated in solid complex (mg)}}{\text{total initial compound in solution (mg)}} \cdot 100$$

$$\text{DL (mg } g^{-1}) = \frac{\text{total compound encapsulated in solid complex (mg)}}{\text{total weight of solid complex (g)}}$$

**Table 4** shows the encapsulation yields (EE) and OA loadings (DL) in different batches of OA/HP-β-CD and OA/HP-γ-CD complexes dehydrated by freeze-drying. As can be seen, the HP-γ and HP-β cyclodextrins showed very similar encapsulation performances, both being a priori suitable for encapsulating OA and producing preparations suitable for testing *in vitro* cell culture. The chemical conformation of cyclodextrins allow stability and protection of the encapsulated drug against adverse physical factors such as temperature. To evaluate this property, we focused on HP-β-type cyclodextrins and measurements were made on lyophilized OA/HP-β-CD complexes stored at different temperatures, with the intention of measuring their stability over time (weeks from complex formation). All complexes were stored under light-protected conditions. The OA load (DL) of each of these complexes was then quantified, with the results shown in **Table 5.** The encapsulated OA loading (LD) data indicate that the OA milligrams per gram of cyclodextrin are hardly modified under the three temperature conditions, even up to 8 weeks after the preparation of the complexes. However, it would be necessary to further test these preparations preserved at different temperatures in functional wound assays on epithelial cells to confirm that despite the fact that no amount of OA is lost, its biological activity is also unaffected and it continues to exert the promoting effect on cell migration.

**Table 4. Complexation performances of OA/HP-β-CD and OA/HP-γ-CD complexes obtained by freeze-drying and Spray Dry**

| | | CD g | OA mg | Solid complexes area HPLC | Solid complexes mg | DL (mg/g) | DL (mg/g) expected | EE (%) |
|---|---|---|---|---|---|---|---|---|
| HP-β-CD | Spray drying | 3,85 | 5,0 | 279,5 | 20,1 | 1,15 | 1,30 | 82,3 |
| | | | | 249,5 | 21,0 | 0,99 | | |
| | | | | 261,5 | 20,3 | 1,07 | | |
| | Freeze drying | 7,7 | 12 | 384 | 20,6 | 1,55 | 1,56 | 97,9 |
| | | | | 373 | 20,4 | 1,52 | | |
| | | | | 391 | 21,4 | 1,52 | | |

| | | CD g | OA mg | Solid complexes area HPLC | Solid complexes mg | DL (mg/g) | DL (mg/g) expected | EE (%) |
|---|---|---|---|---|---|---|---|---|
| HP-γ-CD | Spray drying | 4,4 | 5,0 | 236,8 | 21,4 | 0,92 | 1,14 | 81,5 |
| | | | | 236,0 | 21,6 | 0,91 | | |
| | | | | 234,9 | 20,4 | 0,96 | | |
| | Freeze drying | 8,8 | 15 | 440,4 | 21,4 | 1,71 | 1,70 | 100 |
| | | | | 444,6 | 21,6 | 1,71 | | |
| | | | | 417,8 | 20,4 | 1,70 | | |

**Table 5. Quantifications of freeze-dried OA/HP-β-CD complexes stored at -80°C, 4°C and room temperature protected from light.**

| Weeks after complexation (W) | °C | Freeze dried complexes area HPLC | g freeze dried complexes | DL (mg/g) |
|---|---|---|---|---|
| 0 | 20 °C | 489,0 | 21,3 | 1,11 |
| | 20 °C | 468,9 | 20,1 | 1,13 |
| 1w | (-)80 °C | 527,5 | 22,0 | 1,16 |
| | 4 °C | 517,6 | 21,0 | 1,19 |
| | 20 °C | 415,2 | 19,0 | 1,06 |
| 2w | (-)80 °C | 426,3 | 20,2 | 1,02 |
| | 4 °C | 467,4 | 21,7 | 1,04 |
| | 20 °C | 489,8 | 21,4 | 1,11 |
| 4w | (-)80 °C | 514,0 | 21,9 | 1,14 |
| | 4 °C | 501,0 | 22,2 | 1,09 |
| | 20 °C | 505,0 | 21,9 | 1,12 |
| 8w | (-)80 °C | 447,0 | 20,3 | 1,07 |
| | 4 °C | 431,2 | 19,6 | 1,07 |
| | 20 °C | 459,6 | 20,2 | 1,10 |

The freeze-dried OA/HP-CDs complexes, stored at room temperature, 4°C and -80°C, retained the stability of the encapsulated OA for two months, as the amount of OA was not affected by time and temperature.

Example 2.1: Wound healing scratch assays and protein expression analyses to test OA/CDs complexes activity

In functional scratch assays, OA/CDs complexes are tested for biological activity by quantifying cell migration. The epithelial cell lines Mv1Lu and MDA-MB-231 were used for this purpose. In both cases, cells are grown in culture to form an epithelium that is artificially wounded (time 0 h) and then treated with OA/CDs to determine the optimal concentration at which the greatest stimulatory effect on migration is achieved. A battery of assays was performed to test different concentrations of OA/CDs using both types of cyclodextrins: HP-$\beta$-CD and HP-$\gamma$-CD. Figure 2 shows graphs of the cell migration values obtained with the OA/CDs treatments (migration percentage). In the assays shown, the already optimised concentrations of OA/CDs are indicated after testing different concentrations. In these assays, in addition to the OA/CDs conditions, a control with OA vehicleised with DMSO (OA/DMSO) was included, followed by controls with empty CDs and DMSO. The concentrations of OA/CDs complexes used are given in $\mu$M and refer to the concentration of encapsulated OA and are equivalent to the empty CD controls. An epidermal growth factor (EGF) condition was included as a positive control for cell migration.

Example 2.2: freeze-dried OA/HP-$\beta$-CD and OA/HP-v-CD complexes in scratch assays with Mv1Lu cells

As can be seen in **Figure 2,** both OA complexes with HP-$\beta$ and HP-$\gamma$ cyclodextrins obtained by freeze-dry showed a very significant effect on cell migration at encapsulated OA concentrations of 12.5 $\mu$M and 62.5 $\mu$M, respectively. Both preparations showed cell migration values equal to those of OA/DMSO, which is difficult to handle due to its vehicleisation with DMSO. It is noteworthy that with the OA/CDs complexes, microscopy images showed a higher recruitment of moving cells, with the belt of migrating cells being wider than with OA/DMSO, which would suggest a more effective effect on wound closure. On the other hand, it should also be noted that HP-$\beta$-type cyclodextrins produced the effect on migration at lower concentrations of encapsulated OA than HP-$\gamma$-type cyclodextrins (12.5 $\mu$M vs. 62.5 $\mu$M), thus requiring less of the complex to achieve this effect. This is possibly due to the fact that OA is conjugated to HP-$\gamma$ cyclodextrins with higher affinity (given their higher Kc), causing less OA to be released upon treatment.

Example 2.3: OA/HP-$\beta$-CD complexes dehydrated by freeze-drying and Spray Dry in scratch assays with Mv1Lu cells:

Similarly, following the same design as for the scratch assays, OA/HP-$\beta$-CD complexes obtained by spray drying were tested for comparison with those obtained by lyophilisation (**Figure 3**)**.** It was observed that the complexes obtained by spray drying, despite having a lower dehydration yield than the lyophilized ones, also achieved a significant activity on cell migration compared to the controls. Furthermore, there were no significant differences in cell migration between the two types of preparation and the biological activity of the OA was very similar.

Example 2.4: In vitro assays with OA/CDs complexes; study of marker proteins involved in cell migration by immunostaining

The results of the wound assays were used to study the topographical and subcellular localisation of proteins involved in cell migration. This was done using immunostaining techniques, which allow a topographical measurement of what happens in the wound when OA/CD treatments are added, using specific fluorochrome-labelled antibodies and imaging by confocal microscopy. First, immunostaining for the transcription factor c-Jun, a key factor in cell migration, was performed. Specifically, an antibody against the active (phosphorylated) form of this factor (p-c-Jun) was used to test whether OA/CDs complexes promoted its expression and activation at the wound edges. In addition, co-immunostaining with Hoechst and phalloidin was performed to visualise the nuclei and actin cytoskeleton respectively, giving an idea of the location and structure of the wound (**Figure 4**). Immunostaining for p-c-Jun and analysis of the images showed that, as with OA/DMSO, OA/HP-$\beta$-CD complexes promote the up-regulation of this active form of c-Jun in cells at the edge of the wound. This factor is localised in the nuclei of the cells, where a higher number of p-c-Jun labelled nuclei could be observed than in the basal condition or in the vehicle controls (blank DMSO or CD). To confirm this result and complete this assay, the fluorescence of p-c-Jun (expression) present in the nuclei was quantified using ImageJ image analysis software (**Figure 5**).

Quantification of p-c-Jun fluorescence in the nuclei confirmed that it was much higher with the OA/CDs complexes treatment than with the control, as with the use of OA/DMSO in DMSO. Both OA/DMSO and encapsulated OA promoted the expression and activation of this factor to allow cell migration from the wound edges. However, it should be noted that the effect of the OA/CDs complexes, as seen in the scratch tests, is more effective than that obtained with OA/DMSO. This can be seen in **Figure 5b,** since in the condition with the OA/HP-$\beta$-CD complexes nuclei with high p-c-Jun fluorescence are recorded, a result that is reinforced in **Figure 5c,** where the ratio of activated nuclei to total nuclei was much higher than with OA/DMSO. It is also interesting to note that the effect of OA/CDs complexes on p-c-Jun was higher in the S1 sectors, corresponding to the wound edges, and this effect was lower in the subsequent S2 and S3 sectors, further away from the edge, suggesting a gradient effect on p-c-Jun activation, which was maximal in the edge cells, which are usually found with a higher degree of motility.

On the other hand, the immunostaining technique also allowed us to study the cytoskeletal proteins paxillin and actin. Both proteins are associated and have the function of allowing the assembly and disassembly of the cellular architecture of focal adhesion complexes, which is very important during cell migration and allows cell movement. Their study therefore provides insight into the state of cell dynamics at the wound edge (**Figure 6**).

Paxillin is a protein that forms structures known as foci of adhesion (FAs). These structures anchor the cell to the extracellular matrix and increase in number and decrease in size as the cells move, resulting in a state of cellular dynamization. Therefore, if the encapsulated OA promotes cell migration, the epithelial cells must be in this high state of dynamization. By visualizing the adhesion foci with paxillin and quantifying them using image analysis in imaged, the degree of cell dynamization at the wound edge can be determined. To calculate this degree, the number of adhesion foci is divided by the area of each filopodia present in the cells, i.e. each projection generated by the cells to adhere to the substrate and migrate through the wound space, giving the density of foci per cell (FAs density). On the other hand, the area of each adhesion focus is also calculated to obtain the average size of all the foci. It can be seen that the OA/HP-β-CD complexes, as with OA/DMSO, produce a greater number of adhesion foci and, in addition, they are small foci, all with respect to the basal control and the vehicle control (**Figure 6**). It should be noted that no significant differences were found between OA/DMSO and encapsulated OA in the formation of these structures.

OA/HP-β-CD complexes promoted the remodeling of adhesion sites, as evidenced by the higher number of adhesion sites and their smaller size during migration promoted by OA/HP-β-CD complexes.

Example 2.5: Wound healing scratch assays in MDA-MB-231 cells with freeze dried OA/HP-β-CD complexes

To extrapolate previous results in other epithelial cell model we tested OA/HP-β-CD complexes in MDA-MB-231 cells. We tested several concentrations of OA/HP-β-CD complexes, where MDA-MB-231 cells showed significant cell migration compared to basal (C) and vehicle control HP-β-CD conditions (**Figure 7a and b**). Similar migration percentages values were observed between OA/DMSO and all OA/HP-β-CD concentrations tested with no statistically significant differences reached. Nevertheless, there was a higher migration activity tendency within 17/4.25 $\mu$M/mM and 21/5.25 $\mu$M/mM OA/HP-β-CD concentration complexes. Furthermore, it was easy to notice that, in the case of OA/HP-β-CD, more cells could be found reaching wounded gap area than in OA/DMSO condition (**Figure 7a**).

Example 2.6: In vitro assays in MDA-MB-231 cells with freeze-dried OA/HP-β-CD complexes: key proteins related to cell migration analysis by Western blot

MDA-MB-231 cells are a suitable epithelial model for EGFR expression regulation studies. With the objective of deeply decipher the molecular mechanisms behind OA/HP-β-CD complexes effect on cell migration, we decided to set a whole study of key regulatory proteins involved in cell migration by using sub confluent. Cells were stimulated with 10 $\mu$M OA/DMSO and 21/5.25 $\mu$M/mM OA/HP-β-CD and the activation of different protein was evaluated. Mainly, the phosphorylation consequences of all proteins studied was milder when cells were stimulated with OA/HP-β-CD in comparison to its DMSO solubilized counterpart. To begin with, a p-EGFR stimulation was detected 2 hours after treatment with OA/HP-β-CD, later increasing at 3 and 4 hours, however, when compared to OA/DMSO the stimulation was softer (**Figure 8a and b**). When looking at a downstream EGFR kinase, p-ERK1/2, it was increased by OA/HP-β-CD treatment after 2 hours following the same trend that EGFR phosphorylation (**Figure 8a and b**). Interestingly, OA/HP-β-CD showed significantly lower intensity values at time 2 h, and it was kept higher for longer in time. Regarding p-JNK1/2, both OA/HP-β-CD and OA/DMSO induced stimulation stimulated this kinase at time 1 hours, however later in time dynamics were dissimilar. Interestingly, when blotting total c-Jun and active phosphorylated (p)-c-Jun forms we observed changes on both antigens in response to OA/HP-β-CD, an effect that is evident also in the case of OA/DMSO. In particular, in case of p-c-Jun, the treatment with OA/HP-β-CD showed a mild increase at time 2 h that was sustained up to 6 h. In contrast, OA/DMSO produced a stronger stimulation of p-c-Jun at time 1 hour, it was sustained in time up to 6 hours and exhibited higher values than cyclodextrin complexed OA. In any case, no increases on p-JNK1/2 and p-c-Jun regulatory proteins were observed with DMSO and HP-β-CD vehicle controls. Finally, total c-Jun levels were clearly increased by OA/HP-β-CD with 2 hours delay (3, 4 and 6 h) when compared to OA/DMSO, that stimulated c-Jun overexpression at the early time of 1 h. The overexpression and activation of the above-mentioned proteins, in response to OA/HP-β-CD, imply their participation in the cell migration phenomenon promoted by cyclodextrin-complexed OA.

Example 2.7: Activity of the OA/HP-CD complexes without dehydration on cell migration Mv1Lu cell migration.

OA/CDs complexes need to be dehydrated due to its easier manipulation and addition to cell culture mediums. By contrast, using OA/CDs solutions directly added to the medium can produce osmolarity alterations and contaminations, consequently compromising cell viability and loosing OA activity. We tested liquid OA/HP-γ-CD complexes and freeze-dried OA/HP-γ-CD complexes in wound healing scratch assays by using Mv1Lu cells (**Figure 9**). As expected, liquid OA/HP-γ-CD complexes did not show any significant migration activity, since they had same migration percentages as control (C) condition or vehicle control with empty 0/12.5 μM/mM OPA/HP-γ-CD. A concentration of 0.04 g/ml of the dehydrated complexes was used. Only dehydrated complexes have significant migration activity as indicated in previous assays.

**Claims**

1. A process to complex oleanolic acid that comprises:

   a) putting in contact oleanolic acid and at least one cyclodextrin in an aqueous mixture,
   b) dehydrate the product obtained in the previous step

   to obtain a dry powder comprising oleanolic acid/cyclodextrins complexes.

2. The process according to the preceding claim, wherein the cyclodextrin comprises at least one hydroxypropyl group.

3. The process according to the preceding claim, wherein the cyclodextrin is hydroxypropyl-β-cyclodextrin or hydroxypropyl-γ-cyclodextrin.

4. The process according to any of the preceding claims, wherein the ratio oleanolic acid/cyclodextrin in step a) is between approximately 1:100 to 1:400

5. The process according to any of the preceding claims, wherein oleanolic acid/cyclodextrin are put in contact in step a) for a time comprised between 5 h and 5 days, preferably, 10 h and 3 days, more preferably 15 h and 2 days.

6. The process according to any of the preceding claims, wherein the temperature in step a) is comprised 5°C and 40°C, preferably between 10°C and 30°C.

7. The process according to claims 5 and 6, wherein oleanolic acid/cyclodextrin are put in contact for a time comprised between 15 h and 2 days and a temperature comprised between 10°C and 30°C.

8. The process according to any of the preceding claims, wherein the dehydration is performed by freeze-drying, spray drying, vacuum stove and/or rotary evaporator.

9. The process according to the preceding claim, wherein the freeze-drying comprises subjecting the product of step a) to a temperature of less than -70 °C for an appropriate time, and them under a vacuum, subject said product to a temperature comprised between -60°C and -40°C for at least 2 days to obtain a dry powder.

10. The process according to claim 9, wherein the spray drying comprises injecting the product of step a) into a circuit that sprays said product at a temperature between 110°C and 210°C and then cool it to a temperature between 40°C and 90°C to obtain a dry powder for a time between 10 min and 2 h.

11. A product obtained by the process according to any of the preceding claims 1 to 10.

12. A dry powder comprising a complex of oleanolic acid/cyclodextrin.

13. An aqueous mixture comprising the product of any of the preceding claims 11 or 12 with a concentration of at least 0.01 g/ml.

14. A product obtained by the process according to any of the preceding claims 1 to 10, a dry powder according to claim 12, and/or an aqueous mixture according to claim 13 for use in skin disorders.

15. A product for use, according to the preceding claim, wherein the skin disorders are selected from the group consisting of: wound healing, scarring, eczema, psoriasis, acne, rosacea, ichthyosis, vitiligo, urticaria and/or seborrheic dermatitis

Fig.1

Fig. 2

Fig. 3

**Fig. 4**

**Fig. 5**

Fig. 5 (continuation)

Fig. 5 (continuation)

Fig. 6

Fig. 7

**a**

# p-EGFR/EGFR

Fig. 8

# p-ERK/ERK

# p-JNK/JNK

Fig. 8 (cont)

# p-c-Jun/β-Actin

# c-Jun/β-Actin

Fig. 8 (cont)

Fig. 9

Europäisches
Patentamt

European
Patent Office

Office européen
des brevets

# EUROPEAN SEARCH REPORT

**Application Number**

EP 23 38 2932

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2015 214522 A (NIPPON FLOUR MILLS) 3 December 2015 (2015-12-03) * paragraph [0026] * | 1-15 | INV. A61K9/14 A61K31/201 A61K47/69 |
| A | LÓPEZ-MIRANDA SANTIAGO ET AL: "Complexation between oleanolic and maslinic acids with native and modified cyclodextrins", FOOD CHEMISTRY, vol. 240, 1 February 2018 (2018-02-01), pages 139-146, XP93133537, NL ISSN: 0308-8146, DOI: 10.1016/j.foodchem.2017.07.092 * the whole document * | 1-15 | A61P17/02 |
| X | US 2016/144040 A1 (CHENG JINSHENG [CN]) 26 May 2016 (2016-05-26) | 11-15 | |
| A | * claims 1,9 * | 1-10 | |
| X | CN 102 512 690 A (CHANGCHUN MODERN BIOLOG MEDICINE RES AND DEV CO LTD) 27 June 2012 (2012-06-27) | 11-15 | TECHNICAL FIELDS SEARCHED (IPC) |
| A | * claims 1,8 * | 1-10 | A61K A61P |
| A | WO 2019/054379 A1 (UNIV SHIZUOKA NAT UNIV CORP [JP]) 21 March 2019 (2019-03-21) * example 3 * | 1-15 | |

The present search report has been drawn up for all claims

| Place of search | Date of completion of the search | Examiner |
|---|---|---|
| Munich | 26 February 2024 | Wörth, Christian |

EPO FORM 1503 03.82 (P04C01)

2

## ANNEX TO THE EUROPEAN SEARCH REPORT
## ON EUROPEAN PATENT APPLICATION NO.

EP 23 38 2932

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2024

| Patent document cited in search report | | | Publication date | Patent family member(s) | | | Publication date |
|---|---|---|---|---|---|---|---|
| JP 2015214522 | A | | 03-12-2015 | JP | 6372025 | B2 | 15-08-2018 |
| | | | | JP | 2015214522 | A | 03-12-2015 |
| US 2016144040 | A1 | | 26-05-2016 | CN | 104623681 | A | 20-05-2015 |
| | | | | US | 2016144040 | A1 | 26-05-2016 |
| CN 102512690 | A | | 27-06-2012 | NONE | | | |
| WO 2019054379 | A1 | | 21-03-2019 | JP | 2019052105 | A | 04-04-2019 |
| | | | | WO | 2019054379 | A1 | 21-03-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• *Food Chem*, 2018, vol. 240, 139-46